# EUROPEAN PATENT APPLICATION

(11) **EP 3 958 528 A1**
(43) Date of publication of application: **23.02.2022**
(21) Application number: 20192152.5
(22) Date of filing: 21.08.2020
(51) Int. Cl.: H04L 29/06, H04W 4/021, G16H 40/60

(54) **LOCATION-BASED ACCESS CONTROL OF A MEDICAL ANALYZER**

(71) Applicant: Roche Diagnostics GmbH, 68305 Mannheim (DE); F. Hoffmann-La Roche AG, 4070 Basel (CH)
(72) Inventor: SPRINGER, Thomas, 6390 Engelberg (CH); WINIARZ, Jakub, 6343 Rotkreuz (CH)
(74) Representative: Peterreins Schley

(57) **Abstract**

In a hospital, it may be desired to improve the access control to analytical devices (P1A-P7A) such as blood or saliva testing machines. Accordingly, there is provided a computer implemented method for controlling user access to an analytical device based on a location of a user relative to the analytical device. The method comprises receiving a first location credential of a first user of an analytical device from a location management system (68) of an access controlled facility (8), wherein a first location credential at least partially defines a current location of the first user of the analytical device, updating a permitted user record (PUDB) associated with the analytical device based on a first location credential of the first user, receiving a user logon credential entered into the analytical device as part of a logon process of the analytical device, and permitting a logon to the analytical device if the received user logon credential entered into the analytical device accords with the permitted user record.

## Description

### Technical Field

This disclosure relates to a computer implemented method for controlling user access to an analytical device based on a location of a user relative to the analytical device, and a related apparatus, system, computer program element, and computer readable medium.

### Background

In hospitals, analytical devices of medical samples can be used at, or near to, the point of care in a hospital. Such analytical devices are sometimes designated "Point of Care (POC) testing devices". The analytical devices can communicate a variety of status messages containing information about the technical status of the testing devices with a central server, for example.

A large number and variety of analytical devices may be used throughout a hospital, and with many different grades of user having different training levels present. Controlling access to analytical devices at the point of care is important, to ensure that clinical care standards are met. For example, a medical professional with a given certification should only be permitted to use analytical devices that they have been certified to use, with the aim of improving quality outcomes.

Accordingly, analytical devices and their corresponding control systems may be further improved.

### Summary

According to a first aspect, there is provided a computer implemented method for controlling user access to an analytical device based on a location of a user relative to the analytical device. The method comprises:
receiving a user logon credential of a first user entered into an analytical device as part of a logon process of the analytical device;
receiving a first location credential of the first user of an analytical device from a location management system of an access controlled facility, wherein the first location credential at least partially defines a current location of the first user of the analytical device;
updating a permitted user record associated with the analytical device based on the first location credential of the first user; and
permitting a logon to the analytical device if the received user logon credential entered into the analytical device accords with the permitted user record, as updated based on the first location credential.

An effect of this is that the access control to a specific analytical device is improved. In particular, a permitted user may logon to a specific analytical device, based on the location of that user obtained from a location management system.

Hospitals, and many other access controlled facilities already contain legacy access control systems that report access attempts, and the identity of the users making those access attempts, to a central location (the location management system). Therefore, a data-processing agent or server may be integrated with the location management system at the central location in order to obtain information about the location of users inside access controlled areas with a small amount of equipment retrofit. The approach detailed herein may interface with existing access control systems to improve the logon control to a system of analytical device.

The approach in accordance with the aspects and embodiments discussed herein is difficult to defeat accidentally, or deliberately. Even if a first user of the system analytical devices inappropriately shares their logon credentials with a second user of the system, the requirement to use location information of the first and second users obtained from the access control system, makes the sharing of logon credentials impractical as a method of defeating the logon control approach.

Accordingly, clinical outcomes can be improved, because there is improved control over which users are able to logon to an analytical device. Hence, the certification level and general appropriateness of a user to operate an analytical device can be more effectively controlled, leading to better test result quality, for example.

An assumption of the technique of this specification is that there may be a lower level of certainty that the logon credentials used accurately identify the individual using them at a given analytical device. Accordingly, authentication factors based additionally on the location of an individual as obtained by an access control system enable more accurate control of user access to an analytical device.

According to a second aspect, there is provided an apparatus configured to control user access to an analytical device based on a location of a user relative to the analytical device. The apparatus comprises:
- a communications interface; and
- a processor coupled to the communications interface.

The communications interface is configured to receive a user logon credential of a first user entered into an analytical device as part of a logon process of the analytical device.

The communications interface is configured to receive a first location credential of a first user of the analytical device from a location management system of an access controlled facility, wherein the first location credential at least partially defines a current location of the first user of the analytical device.

The processor is configured to update a permitted user record associated with the analytical device based on the first location credential of the first user.

The processor is configured to permit a logon to the analytical device if the user logon credential entered into the analytical device accords with the permitted user record, as updated based on the first location credential.

According to a third aspect, there is provided a system for controlling user access to an analytical device based on a location of a user relative to the analytical device for analytical device management. The system comprises:
- one or more analytical devices;
- a location management system configured to detect when a user leaves or enters the vicinity of the one or more analytical devices;
- an apparatus according to the second aspect which, in operation, performs the method according to the first aspect, or its embodiments; and
- a communication network configured to communicatively connect the one or more analytical device, the location management system, and the apparatus according to the second aspect.

According to the fourth aspect, there is provided a computer program element comprising computer-readable instructions for controlling an apparatus according to the second aspect which, when being executed by a processing unit of the apparatus, is configured to perform the method steps of the first aspect, or its embodiments.

According to the fifth aspect, there is provided a computer readable medium or signal having stored, or encoded thereon, the computer program element according to the fourth aspect.

Optional embodiments are defined in the dependent claims, to which the reader may now refer, and which are discussed further in this specification.

Certain terms will be used in this patent application, the formulation of which should not be interpreted to be limited by the specific term chosen, but as to relate to the general concept behind the specific term.

As used herein, the terms "comprises", "comprising", "includes", "including", "has", "having", or any other variation thereof, are intended to cover a non-exclusive inclusion.

Although aspects of the technique discussed in this specification are in terms of method steps, the order of several method steps presented is not essential and should be construed in context.

The terms "patient sample" and "biological sample" refer to material(s) that may potentially contain an analyte of interest. The patient sample can be derived from any biological source, such as a physiological fluid, including blood, saliva, ocular lens fluid, cerebrospinal fluid, sweat, urine, stool, semen, milk, ascites fluid, mucous, synovial fluid, peritoneal fluid, amniotic fluid, tissue, cultured cells, or the like. The patient sample can be pre-treated prior to use, such as preparing plasma from blood, diluting viscous fluids, lysis, or the like. Methods of treatment can involve filtration, distillation, concentration, inactivation of interfering components, and the addition of reagents. A patient sample may be used directly as obtained from the source or used following a pre-treatment to modify the character of the sample. In some embodiments, an initially solid or semisolid biological material is rendered liquid by dissolving or suspending it with a suitable liquid medium. In some embodiments, the sample is suspected to contain a certain antigen or nucleic acid.

The term "analytical device" as used herein encompasses any apparatus for obtaining measurement values relating to a medical condition of a patient. In one example, the measurement values may be provided by obtaining a patient sample, and using an analytical device to automatically, or semiautomatically process the patient sample. The analytical device may detect the presence of analytes in the processed sample, from which an assessment of the medical condition of a patient may be made. It is not essential that the analytical device forms the assessment of the medical condition of a patient - for example, a summary of the analytes detected by the analytical device can be provided to a medical professional for further consideration. In another example, an "analytical device" may obtain and process digital data that represents a medical condition of a patient. The digital data may be received as measurement values from other analytical devices, and/or as image, video, or sound data.

In one example, the analytical device may be an analytical device of biological (medical) samples obtained from a patient providing a measurement value relating to a medical condition of a patient. For example, an analytical device may measure light absorption, fluorescence, electrical potential or other physical or chemical characteristics of the reaction to provide the measurement value. Often such patient samples are treated before analytical testing is done. Blood sampled from a patient is e.g. centrifuged to obtain serum or treated with anti-coagulants to obtain plasma.

Analytical testing by an analytical device has, as an example, the goal of determining the presence and/or concentration of an analyte in a patient sample. The term "analyte" is a general term for substances for which information about presence and/or concentration is intended. Examples of analytes are e.g. glucose, coagulation parameters, endogenic proteins (e.g. proteins released from the heart muscle), metabolites, nucleic acids and so on.

Analytical testing by an analytical device configured to analyse patient samples has, as an example, the goal of determining the presence and/or concentration of an analyte in a patient sample. The term "analyte" is a general term for substances for which information about presence and/or concentration is intended. Examples of analytes are e.g. glucose, coagulation parameters, endogenic proteins (e.g. proteins released from the heart muscle), metabolites, nucleic acids and so on. However, obtaining and processing digital data obtained by a camera sensor of a chemical reaction, or an image of the skin of a patient, for example, is another example of analytical testing.

It is not essential that an "analytical device" automatically performs all steps required to obtain data about the medical condition of a patient. For example, some analytical devices may require a POC operator (user) to pipette reagent into a sample in an ampoule or mount a slide prior to the performance of a test. In other cases, the "analytical device" may automatically perform all steps of a sample analysis without operator intervention. In other cases, the "analytical device" may prompt a user to intervene manually at a stage of the analysis.

Alternatively, the analytical device is a handheld or mobile device comprising sensors configured to acquire measurement values from a patient.

An "analytical device" may comprise a portable appliance that can be communicatively connected to a smartphone, tablet PC, smart watch, or other computing device via a USB (TM), Wi-Fi (TM), or Bluetooth (TM) connection, for example. Such a portable appliance may be configured to perform analytical testing by analysing data obtained from one or a combination of sensors.

A measurement value may comprise data collected from, for example, the sensors of a smartphone. By way of example only, a measurement value may be data obtained by a smartphone accelerometer that characterizes a degree of patient tremor. A measurement value may be a photograph of a dermatological condition obtained using a smartphone camera. A measurement value may be a sound recording obtained using a smartphone microphone. A measurement value may be a video obtained using a smartphone for the purposes of assessing patient gait, for example. In this way, standard features of smartphones, tablet PCs, or other computing devices may perform the function of an analytical device. An application executed on a smartphone, or other computing device, is capable of obtaining such data and communicating it to a data processing agent. A wider suite of measurement values may be obtained via an extension device communicatively coupled to the smartphone. For example, an extension device could comprise a digital thermometer.

The term "patient health parameter" as used herein encompasses any aspect of a patient's physiology that is measurable or indicated by an analysis of a patient sample for one or more analyte, or by analysis of data obtained from one or a combination of sensors.

An "analytical device" may be configured so as to be usable in the vicinity of a patient ward, in which case it is often referred to as a "Point of Care (POC) device". However, the techniques discussed herein are not limited to POC devices and may be applied to many types of laboratory analysis system that generate message data.

The term "Point of Care" POC or "Point of Care environment" as used herein is defined to mean a location on or near a site of patient care where medical or medically related services such as medical testing and/or treatment are provided, including but not limited to hospitals, emergency departments, intensive care units, primary care setting, medical centres, patient homes, a physician's office, a pharmacy or a site of an emergency.

In the field of bedside testing or point of care testing, the testing is typically performed by nurses, medical users, or doctors but also pharmacists who are collectively called "operators" herein. However, anyone who possesses the required certification may be an operator. A point of care coordinator POCC may be at the same time an operator of POC analyser(s) and also an operator of POC analyser(s) may be at the same time a point of care coordinator POCC and thus user of portable computing device(s).

The term "point of care testing" POCT as used herein encompasses analysis of one or more items of data provided by an analytical device as defined above, to obtain information about the medical condition of a patient. POCT is often accomplished through the use of transportable, portable, and handheld instruments, but small bench analysers or fixed equipment can also be used when a handheld device is not available - the goal being to collect a patient sample and obtain analytical data in a (relatively) short period of time at or (relatively) near the location of the patient.

In an example, POCT is performed using various analytical devices (POC analysers) such as (but not limited to) analysers for glucose, coagulation, blood gas, urinalysis, cardiac and molecular testing. Results may be viewed directly on the POC analyser(s) or may be sent to the POCT system and displayed in a Laboratory Information System with central lab results, or alongside imaging results in a Hospital Information System.

Therefore, an analytical device may be used in a point of care environment, to perform tests such as (but not limited to) blood glucose testing, coagulation testing, blood gas and electrolytes analysis, urinalysis, cardiac markers analysis, haemoglobin diagnostics, infectious disease testing, cholesterol screening or nucleic acid testing (NAT). Results may be viewed directly on a Point of Care analyser(s) or may be sent to a Point of Care testing system and displayed in a Laboratory Information System with central lab results, or alongside imaging results in a Hospital Information System. The term "patient health parameter" may optionally encompass digital data such as an image or video that provides information about any aspect of a patient's physiology.

In an example, POCT is performed by obtaining digital data such as a photograph of a portion of the skin of a patient, a video of the patient walking, or a sound sample of the patient making a sound.

In an example, POCT is performed using a "portable computing device" that encompasses any electronic appliance that can be moved easily from one location to another, in particular any handheld battery powered mobile appliance, including but not limited to a cellular telephone, a satellite telephone, a pager, a personal digital assistant ("PDA"), a smartphone, a navigation device, a smart book or reader, a combination of the aforementioned devices, a tablet computer or a laptop computer.

The term "point of care device management system" (POC-DMS) as used herein denotes a data processor configured to communicate with and manage or more POC devices via a computer network to enable a POC coordinator to manage the POC devices, or to enable maintenance personnel to monitor the equipment. Optionally, the POC-DMS is a terminal computer connected to the same network that the POC devices are connected to. Optionally, the POC-DMS may be provided as a server, virtual machine or a virtualized server hosted remotely to the network that the POC devices are connected to, enabling remote management of the POC devices. It is not essential that the POC devices (analytical devices) are connected to the same subnet, or network branch, for example, as the POC-DMS.

The term "communication network" as used herein encompasses any type of wired or wireless network, including but not limited to a WIFI, GSM, UMTS or other wireless digital network or a wired network, such as Ethernet or the like. For example, the communication network may include a combination of wired and wireless networks. Analytical device status data may be transmitted over the communication network.

The term "server" encompasses any physical machine or virtual machine having a physical or virtual processor, capable of accepting requests from and giving responses accordingly. It shall be clear to a person of ordinary skill in the art of computer programming that the term machine may refer to a physical hardware itself, or to a virtual machine such as a JAVA Virtual Machine (JVM), or even to separate virtual machines running different Operating Systems on the same physical machine and sharing that machine's computing resources. Servers can run on any computer including dedicated computers, which individually are also often referred to as "the server" or shared resources such as virtual servers. In many cases, a computer can provide several services and have several servers running. Therefore the term server shall encompass any computerized device that shares a resource to one or more client processes. The server can receive, process, and transmit analytical device status data.

The term "server interface" encompasses any hardware-, firmware- and/or software- based module operable to execute program logic to allow communication with an external entity (such as a server or another interface).

The term "data processing agent" refers to a computer implemented software module executing on one or more computing devices, such as a server, that is able to receive analytical device status data from a point of care device, and annotation data from a user, and associate the analytical device status data and the annotation data. The "data processing agent" may be implemented on a single server, or multiple servers, and/or an internet-based "cloud" processing service such as Amazon AWS (TM) or Microsoft Azure (TM). The "data processing agent", or a portion of it, may be hosted on a virtual machine. The data processing agent can receive, process, and transmit analytical device status data.

The term "user interface" encompasses any suitable piece of software and/or hardware for interactions between a user and a machine, including but not limited to a graphical user interface for receiving as input a command from a user and also to provide feedback and convey information thereto. Also, a system or device may expose several user interfaces to serve different kinds of users. The user interface may display graphical elements showing analytical device status data.

A permitted user record is a table, database, or data structure defining which user is permitted to logon to a given analytical device. For example, there is one permitted user record for each analytical device. In an embodiment, the permitted user record of each analytical device may be stored on the analytical device to which it refers. In an embodiment, all, or a subset, of permitted user records in respect of one or more analytical devices may be stored on a server or data processing agent. In an embodiment, the permitted user records are only stored on the server or data processing agent.

Accordingly, a system is proposed that determines a user's access right to an analytical device based on their location in the hospital. Optionally, the access right is also based on the user's certification status. A user's location in the hospital is approximated with the help of and integration with, for example, door and gate access control systems. An example, only when a user is in the access control zone (area) of a given analytical device, will a user list with the username be generated to grant access to the analytical device. When the user leaves the access control zone, the access rights with the user credentials are immediately revoked.

This specification discusses the integration of a data processing agent (for example, executing on a server or a compute cloud) with several databases, and with gate or other access systems (such as badge-based access systems). The data processing agent may access information about the certification status of the user. Through this integration, when one user enters into an access control zone of the hospital where an analytical device is located, their name will be included in a user list generated by the data management system. Optionally, the certification status of the user may also define whether or not the user is entered into the user list.

It is noted that terms like "preferably," "commonly," and "typically" are not utilized herein to limit the scope of the claimed embodiments or to imply that certain features are critical, essential, or even important to the structure or function of the claimed embodiments. Rather, these terms are merely intended to highlight alternative or additional features that may or may not be utilized in a particular embodiment of the present disclosure.

### Description of the Drawings

**FIG. 1** schematically illustrates a networked system for analytical device management according to an aspect.
**FIG. 2** schematically illustrates an example of an analytical device according to an example.
**FIG. 3** schematically illustrates an example of a server configured to host a data processing agent capable of performing a computer implemented method according to the first aspect.
**FIG. 4** schematically illustrates an example of an access plan of a hospital having two floors.
**FIG. 5** schematically illustrates an example of a logical arrangement of a data processing agent, optionally provided on a server or an analytical device, capable of performing a computer implemented method according to the first aspect.
**FIG. 6** schematically illustrates a signalling diagram of location-based access control according to an embodiment.
**FIG. 7** schematically illustrates a further signalling diagram of location-based access control according to an embodiment.
**FIG. 8** schematically illustrates a further signalling diagram of location-based access control according to an embodiment.
**FIG. 9** schematically illustrates a connectivity graph model of the access plan of the hospital illustrated in **FIG. 4****.**
**FIG. 10a** schematically illustrates an example of a user represented in the connectivity graph model at different times.
**FIG. 10b** schematically illustrates an example of a user represented in the connectivity graph model at different times.
**FIG. 11** schematically illustrates a further signalling diagram of location-based access control according to an embodiment.

The figures are not drawn to scale, are provided as illustration only and serve only for better understanding but not for defining the scope of the invention. No limitations of any features should be inferred from these figures.

### Detailed Description

A wide range of different types of Point of Care (POC) analysers (also known as analytical devices) are provided in a healthcare facility, such as a hospital. Blood-gas analysers may be provided close to wards for performing regular assessments of blood gas content, whereas more complicated analytical devices are provided in laboratory or pathology facilities to perform rarer or more complicated test protocols.

It is important to control the access of users to particular analytical devices. In particular, it is important to ensure that a user has been trained and is certified to operate a given analytical device. Furthermore, it is important to ensure that the logon credentials, for example a username, of a user corresponds to the correct identity of the user who is performing tests on a given analytical device, to satisfy traceability and quality control requirements.

A user may forget their logon credentials to an analytical device, and in a pressured environment may ask to borrow the logon credentials of a colleague. Although this is a solution to the problem of needing to logon to an analytical device quickly, such behaviour frustrates the important need to satisfy traceability and quality control requirements of the tests performed. It is preferred that a given user uses their own, unique, logon credentials when logging onto a given analytical device.

The aspects of the solution discussed in this specification exploit the widespread provision of access control systems in hospitals. Hospitals are an example of an access controlled facility. There is already a need to control the access of individuals between different areas of a hospital, and this need has been taken care of with door access control systems, for example. In order to access an area of the hospital through a door, lift, or access gateway of the hospital, a user must satisfy a security challenge. Different users may have different security clearances, for example.

The technique of the present specification is not limited to application in a hospital, and may be applied to other contexts where access control to an analytical device is required in the context of an access controlled system, such as an industrial research laboratory, a military installation, a university laboratory, and the like. According to an embodiment, the technique is for controlling logon to a generic device that requires logon, such as a personal computer or other data terminal.

The security challenge may be provided at an access point from a swipe card system, a PIN entry system, a near field communication (NFC) system, a Wiegand card access system, a facial recognition system, a barcode or QR code scanning system, and many other options. When a user fulfils (or not, as the case may be) the security challenge to obtain access, a location management system is provided with an update of the location of that user. Location management systems are typically in electronic communication with a control system.

In examples, use of an analytical device may need to be restricted to prevent one, or more, of the following cases: (A) to prevent a user logging on to an analytical device that they are certified to logon to, using the credentials of another user, (B) to prevent a user logging onto an analytical device that they are not certified to logon to, using the credentials of another user, (C) to prevent a user logging onto an analytical device that they are not certified to logon to owing to an expiry of a certificate, (D) to prevent a user (whether authorised or not) moving an analytical device to a new location and logging onto the device in the location that it has been moved to, (E) to prevent a user logging onto an analytical device using their own credential and certification, but then leaving the area where the analytical device is located and allowing a second user (whether accidentally or with the knowledge of the first user) to continue using the logon session established by the first user.

Therefore, the location information of a user may be detected by the location management system, interrogated, and used for other purposes, assuming that relevant applicable data privacy standards concerning the use of the location data have been met.

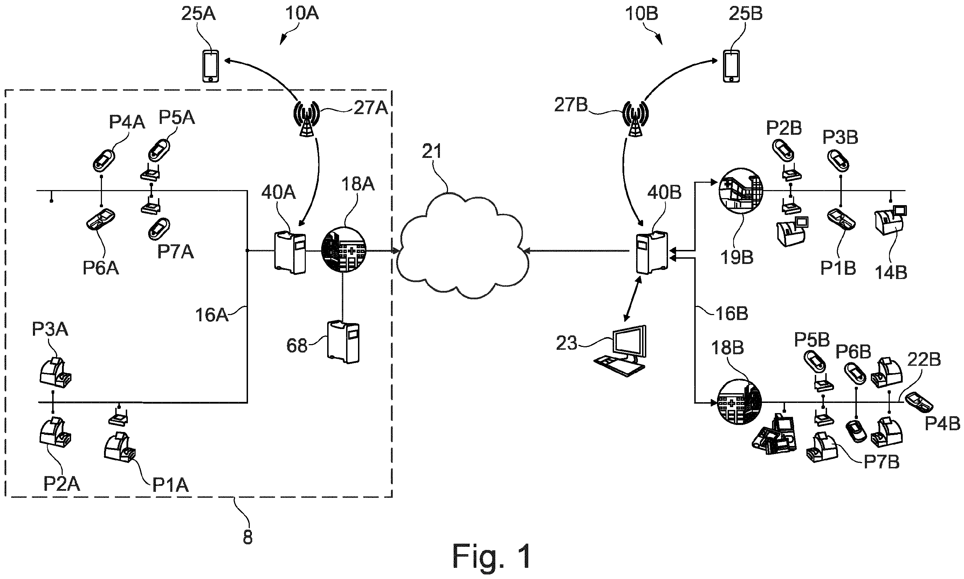

**FIG. 1** schematically illustrates a networked system 10 for analytical device management. The networked system 10 for analytical device management comprises a first network 10A. The first network 10A may be divided into one or more Local Area Networks (LANs) or Wide Area Networks (WANs) corresponding to a location 18A housing analytical devices P1A-P7A. The number of analytical devices in the first network 10A of the networked system 10 is not essential to the functioning of the system discussed herein.

The system comprises one or more analytical devices P1A to P7A, optionally a portable computing device 25A (such as a smartphone), and a server 40A communicatively connected by a communication network 16.

The server 40A may, in an example, host a data processing agent 70 according to the first aspect. In other examples, the data processing agent 70 may be hosted by a cloud computing service distributed over a plurality of servers and computing devices. In particular, the communication network 21 is configured to communicatively couple the one or more analytical devices P1A to P7B.

The communication network 21 may, for example, comprise one or more of a local area network LAN provided over, for example, an ethernet network, a Wi-Fi network, and/or a wide area network WAN such as the Internet. The communications network may comprise a Mobile Telecommunications network 27 such as a 3G, 4G, or 5G system, and/or a hospital PACS network.

Optionally, the network 16A may connect the server 40A directly to the analytical devices (POC devices) P1A to P7B.

Optionally, the network 21 interfaces with an internal communications system 22A of a health facility (hospital) 18A. The internal communications system 22A may be considered to be an intranet, for example. A firewall and other security measures known to a person skilled in the art may be placed in between the internal communications system 22A and the communications network 21 to ensure security and confidentiality. The analytical devices P1A to P7A may communicate with a data processing agent 70 hosted on a server 40, for example, by communicating via the internal communications system 22 and the communication network 16A.

The analytical devices P1A to P7A are provided and configured to analyse one or more patient samples in order to measure one or more patient health parameters. According to disclosed embodiments, analytical devices P1A to P7A may include transportable, portable, and hand-held instruments, but also small bench analytical devices or fixed equipment 14 as well.

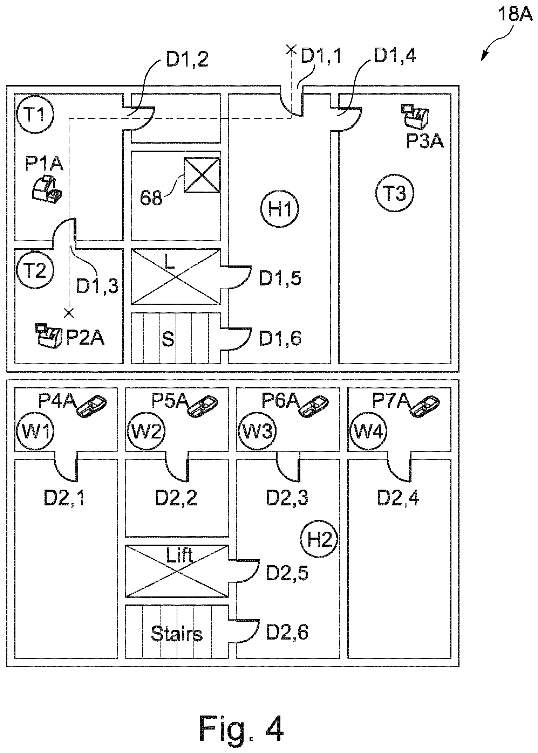

Turning briefly to **FIG. 4****,** the analytical devices P1A to P3A are located on the ground floor of a hospital 18A. As illustrated, analytical devices P1A to P3A may be provided in pathology test laboratories T1-T3. The analytical devices P4A-P7A may be provided in wards W1-W4, respectively, located on a first floor of a hospital 18A.

In order to identify a particular analytical device P1A to P7A, each analytical device is provided with an analytical device identifier code, in particular in the form of an identifier tag such as a barcode and/or an RFID tag or a serial number. Optionally, such identifiers may be associated with an entry in a database of the system for analytical device management.

The networked system 10A for analytical device management further comprises a Point of Care Data Management System (POC-DMS), hosted, for example, on server 40A. The purpose of the POC-DMS is to monitor, and control, one or more analytical devices P1A-P7A in a defined area, or network branch. For example, POC administrator personnel can use the POC-DMS hosted on server 40A to track the condition of one or more of the analytical devices P1A-P7A, to monitor consumable usage, and a wide variety of other management activities.

The networked system 10 for analytical device management also comprises a further network 10B that is illustrated in **FIG. 1****.** The further network 10B represents a network of analytical devices run at a different hospital site, or in a different country, or hospital department as compared to the first network 10B. The description of the individual components provided above in respect of the network 10A also applies to the illustrated components of the further network 10A for reasons of brevity. A skilled person will appreciate that a further network 10B may have a significantly different architecture to that illustrated. The networked system may comprise a remote workstation 23 to enable remote system management, or results monitoring, for example.

The networked system 10A for analytical device management is installed within an access - controlled location denoted by a dotted line 8. Furthermore, a location management system 68 is communicatively coupled to the network 10A to enable location information of users and analytical devices to be obtained, including for example, information about when a user passes through an access-controlled door or uses an access-controlled lift, or a security barrier.

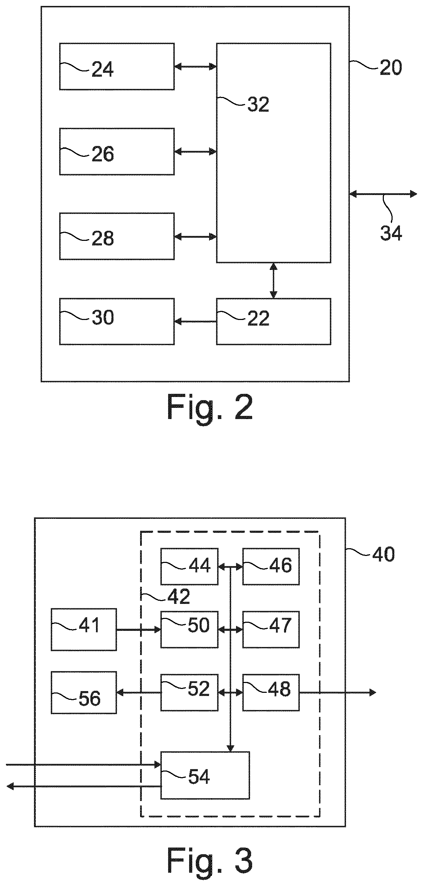

**FIG. 2** schematically illustrates an example of an analytical device 20 (Point of Care (POC) device).

The example of the analytical device 20 comprises a power supply 22 configured to provide power to the analytical device 20. The power supply 22 may be, for example, a lithium ion battery enabling the analytical device 20 to be portable, or a mains power supply. The power supply 22 provides electrical energy to the other elements of the analytical device 20. The other elements comprise, for example: a sensor device 24, an electromechanical subassembly 26, a specimen processing section 28, and an analysis unit 30. A control and communication subsystem 32 interfaces with the previously listed modules. A communications link 34 enables data transfer to and from the analytical device 20.

The sensor device 24 may, for example, comprise a photometer for measuring optical transfer characteristics through a fluid sample, although many other types of sensor could be used dependent on the application of the analytical device 20.

The electromechanical subassembly 26 is configured to receive sample ampoules or cassettes and load them into a specimen processing section 28 so that they can be analysed by the sensor device 24. Following analysis, the electromechanical subassembly 26 may eject the sample ampoules or cassettes.

The specimen processing section 28 may perform pre-analysis functions such as agitation or heating of the sample to a required analysis temperature.

The analysis unit 30 may receive data from the sensor device 24 comprising a characterization of a specimen contained in the specimen processing section 28. The analysis unit 30 may perform one or more data processing operations on the data from the sensor device 24. For example, the analysis unit 30 may ensure that the result from the sensor device 24 is within expected boundaries.

Following analysis, the analysis unit 30 may transmit data from the sensor device 24 via the communications and control unit 32 to the system for analytical device management via the communications network 21, and eventually to a data processing agent 70 hosted on, for example, a server.

A skilled person will appreciate that the foregoing description of an analytical device 20 is provided for illustrative purposes, and that practical analytical devices may comprise fewer or more modules and functionalities. In particular, the electromechanical subassembly, the sensor device 24, and the specimen processing section 28 are not essential. In particular, the analytical device 20 may comprise sensors such as a camera or a microphone, and the analysis unit may receive image, video, or sound data, for example. In an example, the analytical device 20 is configured to receive data from, for example, the camera or microphone and to analyse data for medically relevant indications.

In an embodiment, the control and communication subsystem 32 is configured to host a Permitted User Engine 82 (PUE) and/or a permitted user database (PUDB). The composition of a PUE 82 and the PUDB will be discussed subsequently. In brief, the PUE 82 and/or PUDB enable user access to an analytical device 20 P1A-P7A to be controlled, based on the location of the user relative to the analytical device. Although the functions of the PUE 82 and/or PUDB may be performed by a data processing agent 70 executing on a server 40, it may be preferable to perform the functions of the PUE 82 and/or PUDB on an analytical device 20, to minimise the logon latency onto the analytical device.

In an embodiment, the control and communication subsystem 32 of the analytical device 20 is communicatively coupled to, for example, a certificate database 60, a user database 62, an analytical device database 64, a building information management database 66, and a location management system 68, for example. A data processing agent executing on an analytical device P1A-P7A may be able to interface directly with the one or more databases, and not require a separate server 40 to implement the data processing agent 70.

**FIG. 3** schematically illustrates an example of a server 40 (apparatus) configured to host a data processing agent.

In this example, the server 40 comprises a motherboard 42 comprising a random access memory 44, a read-only memory 46, a processor 47, an input/output interface 48, a data storage interface 50 (such as an interface to a non-volatile memory 41), a display interface 52, and a communication interface 54, however a skilled person will appreciate that many different types of server configuration can be provided with more or fewer modules having other functionality.

The processor 47 of the server 40 is configured to obtain, from an interfaced non-volatile memory 41 (for example), computer readable instructions which, when executed, instantiate a data processing agent for controlling user access to an analytical device based on a location of a user relative to the analytical device, as defined by the computer implemented method according to the first aspect, or its embodiments.

A data processing agent 70 is instantiated on the server 40 from machine-readable instructions obtained, for example, from the random-access memory 44, or the read-only memory 46, the input/output interface 48, or the data storage interface 50.

Optionally, the server 40 hosting the data processing agent 70 is configured to display a location of an analytical device PI A - P7A, and/or the location of at least one user, to a user on a local display via a local display driver 56, or by communicating the inferred condition to a further device such as a smart phone 25A.

In an embodiment, the server 40 (and the data processing agent 70 executed thereon) is communicatively coupled, via the communication interface, 54, to, for example, a certificate database 60, a user database 62, an analytical device database 64, a building information management database 66, and a location management system 68, for example. In an embodiment, the server 40 (and the data processing agent 70 executed thereon) is communicatively coupled to a plurality of analytical devices P1A - P7A.

It is not essential for a server to be provided as a single computational device. For example, in an embodiment, the functions of the data processing agent 70 may be shared between a plurality of servers, and/or a cloud computing service such as Microsoft Azure (TM) or Amazon cloud (TM), for example.

FIG. 4 schematically illustrates a floorplan of a hospital 18A having two floors. It will be appreciated that the floorplan of the hospital is provided as an example, and that many other hospital designs could be used according to the techniques discussed herein.

The ground floor of the hospital comprises a hallway HI and three pathology facilities T1-T3. T3 comprises analytical device P3A and is accessible by access point D1,4. The pathology facility T1 comprises analytical device P1A, and is accessible by access point D1,2. The pathology facility T2 comprises analytical device P2A and is accessible via pathology facility T1 and the access point D1,3. The ground floor of the hospital also comprises a lift L and a stairwell S to a first floor. The lift is accessible via access point D1,5. The stairwell is accessible via access point D1,6. At least one of the access points may comprise an access control device.

For example, the access control system may provide access control devices such as a swipe or RFID card access system, an iris scanning system, a QR or barcode based access system, a Wiegand access system, a PIN access system, a photo-ID system, an elevator control system, and/or a wireless networking tracking system.

The upper floor of the hospital is entered from the stairwell via access point D2,6 or via the lift D2,5 into the upper hallway H2. From upper hallway H2, four patient wards W1-W4 may be accessed via respective access points D2,1 - D2,4. Each respective patient ward W1-W4 contains an analytical device P4A-P7A.

The location management system 68 is communicatively coupled to at least one of the access points, and preferably to all of the access points, and other location tracking means. The location management system 68 is configured to receive signals comprising at least an identifier of an individual aiming to progress through the access point. Whether, or not, an individual is allowed through the access point is defined by a local access control policy hosted by the location management system 68. Even if an individual presents an identifier to an access point and is denied passage through the access point, the location management system 68 may log such an attempt

In an embodiment, the time at which identifier is presented to an access point may be logged. Accordingly, the location management system 68 may construct a detailed overview of the personnel registered in the location management system 68 who are present in the hospital at a given time, and may be able to localise the presence of registered users based on the access points that they present their identifier to, and optionally the average time that they take to travel between locations.

According to a first aspect, there is provided a computer implemented method for controlling user access to an analytical device P3A based on a location of a user relative to the analytical device. The method comprises:
receiving 72 a user logon credential of a first user entered into an analytical device P3A as part of a logon process of the analytical device P3A; and
receiving 74 a first location credential LTE of the first user of the analytical device P3A from a location management system 68 of an access controlled facility 8, wherein the first location credential LTE at least partially defines a current location of the first user of the analytical device;
updating 76 a permitted user record PUDB associated with the analytical device P3A based on the first location credential LTE of the first user; and
permitting 78 a logon to the analytical device if the received user logon credential entered into the analytical device P3A accords with the permitted user record PUDB.

Typically, the user logon credential is a username such as an alphanumeric string as mandated by the proprietary standard of an analytical device manufacturer, although any other formats enabling unique identification of individual can be used.

The logon credential may be entered into the analytical device P3A using a keypad, a graphical user interface, and the like. Advantageously, infection control may be improved if a logon credential can be transferred to an analytical device without physical contact, and thus the user logon credential may be transmitted using an NFC protocol, RFID protocol, a QR code or barcode, and the like. Optionally, the logon credential may be entered into a computer device in proximity to the analytical device P3A. The function of the logon credential is to uniquely identify one user of the analytical device P3A.

Usually, the logon credential is split into two parts - a username in the form of, for example, an alphanumeric string is entered. The analytical device P3A may challenge the user to enter a secret password. The password may be generated in accordance with a password policy. Furthermore, the system responsible for holding and protecting the passwords may be provided in accordance with industry-standard password protection approaches. Optionally, the logon credential may be obtained via a two-factor authentication process.

In an embodiment, the login process and password check employ a password hashing approach so that the user database 62 does not store full passwords, but cryptographically hashed versions of user passwords. For ease of presentation, and because cryptographic hashing is not the focus of this specification, password hashing is not illustrated or described herein, however a skilled reader will appreciate that it may be used in accordance with techniques of this specification.

The term "location credential" (or location credential data) defines that prior information about the present location of one or more users is also a factor in the granting, or not, or permission to use the analytical device P3A, in addition to the user logon credential. The location credential may be in many different formats. A minimum standard for the location credential is that it should be possible to verify when a valid user of an analytical device is in the same access control zone (T3, for example) as the analytical device itself (P3A, for example). An access control zone may be a room with access control doors D1,4 into, and out of, the access control zone.

It is not essential that the location credential identifies the location of the user to a given number of metres, or a grid reference, and the like. Furthermore, it is not essential the location credential identifies the valid user to a specific room. For example, security may be enhanced if the location credential is able to define that the user is not in a given subset of the access control zones (rooms) of the hospital, for example.

Optionally, the "location credential" is the location of at least one user within at least one access control zone T3 of an access-controlled facility 8. Optionally, the "location credential" is the location of at least one user within a subset of access control zones, selected from a set of access control zones, of an access-controlled facility.

Optionally, the network of access control zones may be modelled using a directed graph representation, with edges of the graph representing access control doors, and vertices of the graph representing access control zones (rooms) comprising at least one analytical device. However, it is not essential that a directed graph representation is used to model the accessibility of analytical devices, and similar functionality can be modelled in a standard database, for example. However, a directed graph representation of an access control scheme, optionally compiled from a building information model, may enable a reduced latency of computation when searching a large access control network, for example.

Updating 76 a permitted user record PUDB comprises removing or adding to a database (table) comprising a unique identifier of a user who is permitted to use an analytical device P3A. Optionally, the database PUDB is hosted by a data processing agent 70 that is hosted on a server 40 which may be geographically remote from the analytical device P3A. This makes a centralised overview of access control policies easier to obtain.

In another option, the database PUDB is hosted on a specific analytical device P3A. In this case, logon latency may be reduced to a minimum, because the table of permitted users of the analytical device P3A is hosted on the analytical device itself, and no high-latency network lookup operations are required before enabling a user to logon to the analytical device P3A.

In an embodiment, when stored locally on a specific analytical device P3A, the local permitted user record PUDB(P3A) on a given analytical device P3A stores the logon credentials of users who have relevant location credential. In this case, the PUDB(3A) stores location credentials of a user in room T3, for example.

The data processing agent 70 may maintain a location tracking engine LTE of users in different access control zones of a hospital. The data processing agent 70 may provide ("push") logon credentials to the local permitted user record PUDB(P3A) of an analytical device P3A when a permitted user enters the same access control zone that hosts the analytical device P3A. When the data processing agent 70 detects that a permitted user has left the access control zone that hosts the analytical device P3A, the data processing agent 70 may remove ("pull") logon credentials from the local permitted user record PUDB(P3A) of an analytical device P3A. Therefore, location credentials obtained via a location management system 68 may be used to continuously update local permitted user records held at each analytical device P1A-P7A.

If the logon credential presented to the analytical device P3A (for example, the combination of username and password) accords with (belongs to) a user in the access control zone of analytical device P3A, user logon to the analytical device P3A is permitted. This allows a valid user to access the functions, or a subset of the functions, of the analytical device P3A to, for example, analyse a biological sample taken from a patient to identify for a biomarker indicative of a medical condition.

If logon credential presented to the analytical device P3A does not accord with the location of a valid user, then logon to the analytical device is denied, and optionally the log files of at least a user database 62 and/or an analytical device database 64 are updated to make a record of the attempted erroneous access. For example, if a logon credential is used that belongs to a valid user, but that valid user is, at the instant of logon to the analytical device P3A, in an access control zone that does not contain the analytical device P3A, logon to the analytical device P3A will be denied.

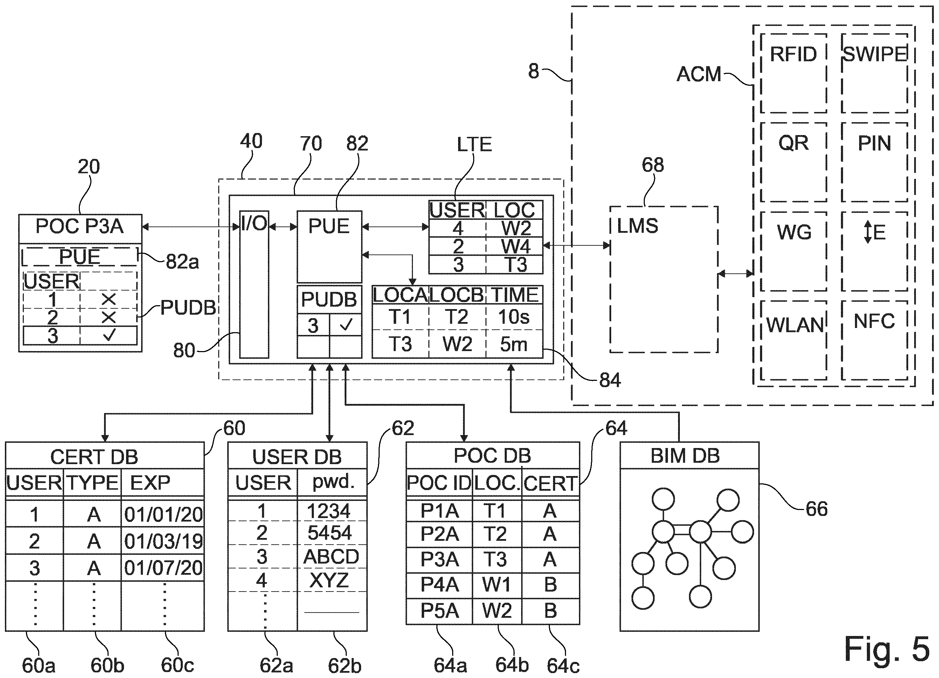

**FIG. 5** schematically illustrates an example of a logical arrangement of a data processing agent 70, optionally provided on a server or an analytical device, capable of performing a computer implemented method according to the first aspect.

A skilled person will appreciate that the presence or absence of a graphical connection between elements in **FIG. 5** is not limiting, and that the example of **FIG. 5** is intended to show one approach as to how databases, an analytical device, and a location management system could be integrated. Other topologies are possible, without departing from the teaching of this specification.

**FIG. 5** illustrates an analytical device P3A 20 as already discussed in association with **FIG. 3** previously, for example. The analytical device P3A is communicatively coupled to a server 40 configured to execute a data processing agent 70.

In particular, the data processing agent 70 comprises a data I/O handler 80 comprising, for example, a subroutine enabling communication with external databases, one, or more, external analytical devices P1A-P7A, a networked system 10A and an external location management system 68.

The data I/O handler 80 is communicatively coupled to a permitted user engine 82.

The purpose of the permitted user engine 82 is to interact with a plurality of databases, and the external location management system 68, to derive one, or more, permitted user records PUDB that may then be associated with a given analytical device P3A.

Optionally, the data processing agent 70 may push one, or more, permitted user records PUDB to a relevant analytical device P3A. This enables user authentication at a relevant analytical device P3A to be performed accurately, but with minimal latency.

Optionally, a relevant analytical device P3A may poll the data processing agent 70, to obtain a permitted user record PUDB held in respect of the relevant analytical device P3A by the data processing agent 70. This enables accurate user authentication at P3A and enables the data processing agent 70 to maintain an overview of the authentication status of an entire system 10A of analytical devices P1A-P7A.

Optionally, the permitted user records of P3A are both maintained both at the data processing agent 70, and are pushed to the relevant analytical device P3A. Optionally, the permitted user records pushed to the relevant analytical device P3A, or hosted by the data processing agent 70, are refreshed at a time interval that is short, compared to the time taken to walk or run through a location in a host hospital. This ensures that the permitted user database held either on the data processing agent 70, or the analytical device P3A is coherent, and accurately, reflects the location of personnel in the host hospital.

Optionally, the permitted user records of P3A are updated asynchronously. In other words, as soon as an access control point identifies a change in location of a user between access control zones in the hospital, an asynchronous update of the user's location is sent to the data processing agent 70, to update the permitted user record PUDB. This ensures that the permitted user record PUDB is based on timely location information represented in the location tracking engine LTE.

Optionally, the permitted user engine 82 may be hosted by an analytical device P3A, without requiring a server 40. Modern microprocessors are capable enough to execute the permitted user engine 82 as a background process in an analytical device P3A equipped with a communications interface that is able to communicate directly with the databases and the location management system 68.

The data processing agent 70 comprises a location tracking engine LTE. The location tracking engine LTE obtains location information from a location management system 68. The purpose of the location tracking engine LTE is to provide a representation of the location of each registered user of the analytical device system present within the access-controlled facility 8. The term "location" may be taken to mean an access-controlled zone of a building plan, for example.

A basic implementation of the location tracking engine LTE may comprise a plurality of records, in which one record connects a unique user identifies, and also an access control zone within an access controlled facility 8 that the user associated with the unique user ID was last identified in, by the location management system 68.

Optionally, a more advanced implementation of the location tracking engine LTE may model the access-controlled facility 8 as, for example, a directed graph. The access-controlled locations may be represented as vertices of the directed graph. Access control points between at least two access-controlled locations may be represented by edges of the directed graph.

A location tracking engine LTE based on a directed graph may be compiled, for example, from a building information management database 66. A building information management database is a centralised repository of relevant building relevant data such as the location of water pipes, emergency building escapes, electrical lines, store cupboards, fuse boards and the like. A building information management database will often also contain information about the location control or location management points and doorways, stairwells, security access points, and lifts inside a building. Therefore, a building information management database may be automatically parsed to generate a directed graph for modelling access control within the hospital. A building information management record is written in a code-like format (for example, using XML), may, thus, be compiled into a graph.

Modelling the hospital as a directed graph has several advantages. A first user may be assigned to a first access zone security classification. A second user may be assigned a second access zone security classification. A first room may be provided with a first security classification. A second room may be provided with a second security classification. A first user may be permitted to access the first room, but not the second room. A second user may be permitted to access the second room, but not the first room. A second user may be permitted to access both the first and second rooms. The population of users of the system may have a heterogenous or a homogeneous access control zone profile.

When generating a permitted user record PUDB using the permitted user engine 82, security credentials of each user, and each access control zone, may be used to simplify the computation of the permitted user database of each access control zone, for example.

Optionally, the data processing agent 70 comprises a location time index 84. The location time index 84 comprises, for example, a row representing an access control zone in the access control location 8, and at least one column representing an access control zone in the access control location 8. Each cell in the location time index 84 contains an estimate of the time taken for a user to move from the access control zone denoted in the row reference of the table, for user to move to the access control zone denoted in the column reference of the table.

It is not essential that the location time index 84 reproduces all time estimates between all locations. For example, estimates on the diagonal of the location time index table will always be zero. Many other routes in the location time index 84 will not, in fact, exist in real life because no corridor will exist connecting such locations in the hospital. Accordingly, the location time index 84 may be a sparse matrix.

A function of the location time index 84 is to enable the permitted user engine 82 to identify whether a user has validly moved from a first location in the hospital to a second location in the hospital by ambulating through corridors, stairs, lifts, and other access points of the hospital. The first user may desire to be logged onto the analytical device P3A as quickly as possible. The first user may telephone or email a colleague (second user) in another location of the hospital who also has permission to use the analytical device P3A. By consensus, or by social engineering, the first user may obtain from the other colleague their logon details to the analytical device P3A or to the analytical device management system POC-DMS.

In such a case, the data processing agent 70 enables the permitted user engine 82 to interrogate the location time index 84 when receiving the logon credentials of the second user at analytical device P3A. The data processing agent 70 may identify from the location tracking engine LTE that the second user is not in the same location as the analytical device P3A that the first user is attempting to use and deny the logon.

Alternatively, the data processing agent 70 may identify from the location time index 84 that the last-known location of the second user is a given ambulation time away from the analytical device P3A that the first user is trying to access, where the ambulation time is defined in the location time index 84. The data processing agent 70 may forbid logon attempts to the analytical device P3A using the second user's logon credentials if they occur within an amount of time that is less than the amount of time that it would take the second user to ambulate to the location of the analytical device P3A.

This may solve a problem that in some hospitals, departments are spaced apart in a variety of small buildings with fewer access control points separating the buildings, leading to poorer location resolution of users. By forbidding logon attempts using the second user's logon credentials for the amount of time that it would take to walk to the analytical device P3A, the first user may be deterred from attempting to acquire the logon details of the second user, but the second user is not inconvenienced when they attempt to use analytical device P3A legally.

Optionally, the location management system is not a part of the first aspect but is third party access control system to which the data processing agent 70 is communicatively coupled.

The data processing agent 70 is communicatively coupled to a user database 62. The database comprises a plurality of records. Each record of the plurality of records comprises at least a user identification field 62a, and a user authentication field 62b. The user authentication field 62b may not store a plain password but may also store authentication data in the form of a password hash, for example. Of course, the user database 62 may contain many more field types required by a typical a point-of-care management system (POC-DMS).

The data processing agent 70 is communicatively coupled to an analytical device database 64. The purpose of this database is to store a record of the access control zone that analytical device P1A-P7A resides within, for example. Therefore, the analytical device database 64 comprises a first set of fields 64a comprising analytical device identifiers P1A-P7A.

The analytical device database 64 comprises a second set of fields 64b comprising a present access control zone of the analytical devices P1A-P7A. In the case of immobile or bench-top analytical devices, the known location of the analytical device may be permanently set in the analytical device database 64 by a POC system manager. However, many analytical devices P3A are portable and may be carried around by hand, or on a trolley. Accordingly, the POC system manager may update fields of the analytical device database 64 in respect of mobile devices, to define which access control zones a given analytical device P3A is moved into.

Of course, more advanced techniques for tracking the location of a portable analytical device P4A-P7A may be used. A portable analytical device P4A-P7A may be identifiable on the network 10A owing to a network address, a MAC address, a firmware version number, and the like. Accordingly, no movement of a subset of analytical devices P4A-P7A may be automatically updated in the second set of fields 64b of the analytical device database 64, as the devices are moved between access control zones of the hospital.

It is not essential that the analytical devices P1A-P7A are static in one access control zone. The method according to the first aspect can still be applied if one or more analytical devices P1A - P7A are translated from a first access control zone to a second access control zone, because the analytical device database 64 may track a current access control zone of the one or more analytical devices P1A-P7A as they are moved. In turn, this means that it is still possible for a location credential of a user to be compared to the access control zone of the one or more analytical devices P1A-P7A as they are moved around.

The analytical device database 64 may comprise a set of fields 64c corresponding to analytical devices P1A-P7A in the system 10A. The set of fields 64c define, for each analytical device P1A-P7A, one or more certificates that are required to logon to each analytical device.

The data processing agent 70 is communicatively coupled to a certification database 60. The certification database 60 comprises a plurality of records arranged by registered user 60a of the POC system. For each registered user 60a of the system 10A, a plurality of type records 60b define, for each registered user 68 of the POC system, which certificates the user possesses relevant to the operation of the analytical devices P1A-P7A present in the system 10A.

Optionally, the schema of certifications used to define the certifications in the plurality of type records 60b of the certification database 60 is the same as the scheme of certifications used to define the certificates that are required to logon to the given analytical device in the set of fields 64c of the analytical device database 64.

Optionally, the certification database 60 comprises, for each user record 60a and/or each certificate type associated with a user record in the certification database 60, an expiry date field 60c. Optionally, the permitted user engine 82 may be configured to deny a logon to a given analytical device P3A if the user is attempting to logon to a given analytical device P3A logon with an expired certification.

The data processing agent 70 is communicatively coupled to a location management system 68. The provision of the location management system 68 is not essential to the apparatus of the second aspect or its embodiments, because typically an access controlled facility 8 already comprises a location management system 68 that an apparatus according to the second aspect may interface with.

Optionally, the data processing agent 70 is configured to perform data transformation to enable user location data, in a data format provided by the location management system, to be utilised by the data processing agent 70, enabling identification of the location of one or more registered users of the system 10A.

For example, the user identification indexes of the users registered in the location management system will usually not match the user identifier of the system 10A as defined in field 60a of the user database 62, because the external location management system is a legacy system. Therefore, the data processing agent 70 may be configured to transform, or to convert, the user identification indexes of the users registered in the location management system 68 to a format that can be indexed to the user database 62.

The location management system 68 is communicatively coupled to one, or a plurality, of access control modalities in the hospital 18A. The access control modalities enable the location management system 68 to infer, or to detect, when a unique user is present, or has been present, in at least one access control zone of the access-controlled facility 8.

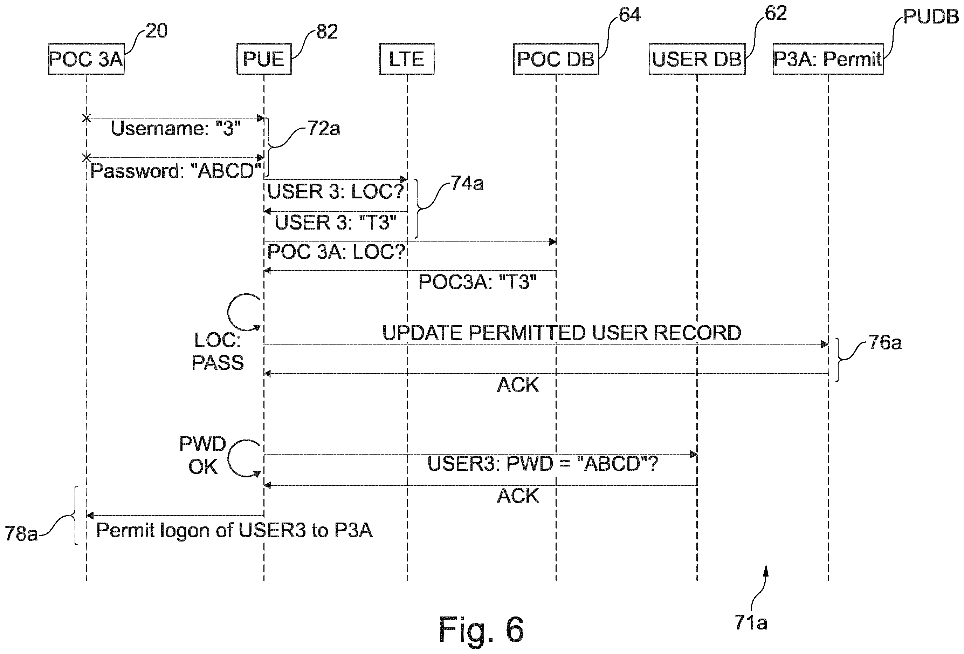

**FIG. 6** schematically illustrates a signalling diagram of location-based access control according to an embodiment. In particular, **FIG. 6** illustrates a successful logon to an analytical device based on a permitted user record PUDB. The order of signalling shown is exemplary, and certain input signals may be received in a different order. The location of devices in the examples are given with reference to the floor plan of **FIG. 4****.**

For example, the row of boxes at the top of **FIG. 6** correspond to like-named or labelled items in **FIG. 5****.** Initially, user logon credentials are transmitted from the analytical device P3A and received 72 by the server 40 (data processing agent 70), optionally by the permitted user engine 82. The user logon credentials may comprise a username and a password, although many other logon credentials may be used that uniquely identify an individual. In an example, an assumption is that there may be a lower level of certainty that the logon credentials used accurately identify the individual using them at a given analytical device P3A.

The permitted user engine 82 queries the location tracking engine LTE with the identifier of the user attempting to logon to the analytical device P3A. The permitted user engine 82 receives from the location tracking engine LTE a location of the user within the access control zone 8. If a present location of the user within the access control zone 8 cannot be found, the last-used location may be used.

Alternatively, an exception handling routine may be initiated, because the absence of a record defining the location of the user entering location credentials into the location tracking engine LTE implies that the valid user of the analytical device is not present in the access control zone, and that logon credentials have been misappropriated.

The permitted user engine 82 queries the analytical device database 64 to identify the present location of analytical device P3A. The analytical device database 64 responds with the present (or, in an example, last known) location of analytical device P3A.

In this case, the permitted user engine 82 identifies a match between the access control zone "T3" of the analytical device P3A, and the location of the user "3" in access control zone "T3". Therefore, the permitted user engine 82 can conclude that a user "3" presenting a user credential to analytical device P3A is genuine. The permitted user engine 82 updates 76 the permitted user record PUDB, and optionally receives an acknowledgement from P3A.

Optionally, the permitted user engine 82 may execute on a data processing agent 70, and maintain a copy of the permitted user record PUDB of P3A on an analytical device P3A. Alterations to the permitted user record PUDB may be pushed or pulled to the analytical device P3A. This enables significant improvement in logon latency at an analytical device P3A, whilst retaining the security advantages of the present technique.

The permitted user engine 82 interrogates the user database 62 to verify the user logon credential entered into the analytical device P3A.

If the user logon credential is correct, and the user to which these logon credential relates is present in the permitted use record PUDB, the logon is successful and the data processing agent 70 permits logon of a user to the analytical device P3A.

If the logon credential is incorrect, or the user to which the logon credential relates is not present in the permitted user record PUDB, then a logon to the analytical device P3A is not permitted.

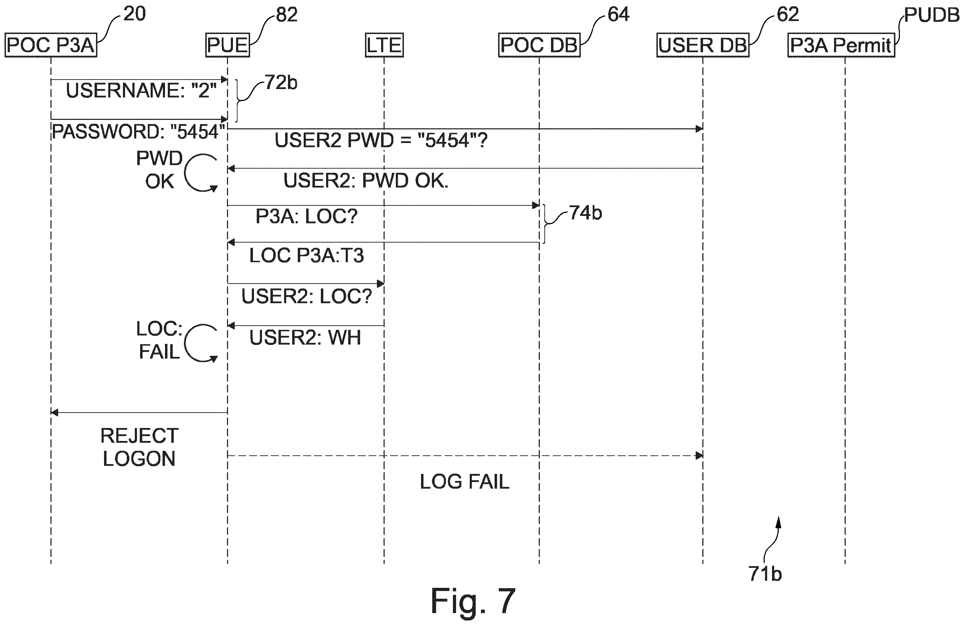

**FIG. 7** schematically illustrates a signalling diagram of location-based access control according to an embodiment. In particular, **FIG. 7** illustrates an unsuccessful logon to an analytical device based on a permitted user record PUDB owing to a deficiency in location credential data. Like steps and processes are not repeated to aid brevity and may be taken from the description of the foregoing embodiment.

For example, the scenario of **FIG. 7** illustrates a case in which an unauthorized user has acquired the logon credentials of user "2". In other words, the permitted user engine 82 interrogates the user database 62 and concludes that a permitted user ID and password and user in respect of P3A has been supplied.

The permitted user engine 82 interrogates the analytical device database 64 to obtain the present, or last-known, location of the analytical device P3A.

The permitted user engine 82 interrogates the location tracking engine LTE to identify the present, or last-known location of user "2" in the access-controlled facility 8. The location tracking engine LTE returns the result that user "2" was last known, or is present, in access control zone "W4" of the hospital. The permitted user engine 82 compares the present (or last-known) location of user "2" returned from the location tracking engine with the present (or last-known) location of the analytical device P3A to which a logon credential for user "2" has been supplied. The permitted user engine 82 concludes that the location of user "2" and the location of analytical device P3A do not match.

Accordingly, the permitted user engine 82 does not enter the user "2" into the permitted use record PUDB of P3A. Because the user logon credential does not accord with the permitted user record 82 (because there is no record of the user "2" in the permitted use record 82), the permitted user engine 82 rejects the attempted logon of the unauthorized user onto analytical device P3A.

Optionally, a log entry may be entered into the user database 62, or the analytical device database 64, for example, optionally, an alarm message may be transmitted to an analytical device management system P3A. Optionally, an audible or visual alarm may be provided at the analytical device P3A to which the unauthorized user is attempting to gain access, to attempt to discourage non-compliant behaviour.

According to an embodiment, the computer-implemented method further comprises:
detecting, via the location management system 68, that the first user has left a controlled area containing the analytical device P3A based on a second received location credential; and
removing the first user from the permitted user record PUDB of the analytical device.

According to an embodiment, the permitted user record PUDB is hosted by the analytical device, and the method further comprises updating the permitted user record PUDB to define that the first user is permitted to logon to the analytical device based on the location credential of the first user.

According to an embodiment, the permitted user record PUDB is hosted by the analytical device, and the method further comprises deleting the first user from the permitted user record PUDB of the analytical device based on the location credential of the first user.

According to an embodiment, the permitted user record PUDB is hosted by the data processing agent 70, and the method further comprises deleting the first user from the permitted user record PUDB based on the location credential of the first user.

According to an embodiment, the permitted user record PUDB is hosted by the data processing agent 70, and the method further comprises deleting the first user from the permitted user record PUDB based on the location credential of the first user.

According to an embodiment, the data processing agent 70 obtains from the location tracking engine, for example by polling, updated user location information on a regular basis.

According to an embodiment, the data processing agent 70 updates the location tracking engine LTE according to an event-based signalling scheme. For example, when a location of a user reported by the location management system 68 (external to the data processing engine 70) changes, this is an event, and the location tracking engine LTE updates its user location table accordingly.

The data processing agent 70 obtains, from the analytical device database 64, updated analytical device location data 64b on a continuous, polled, or event-triggered basis. The permitted user engine 82 continuously, or at sampling intervals, compares, for each analytical device P1A-P7A connected to the network 10A, the analytical device location data 64b to the user location information from the location tracking engine LTE. The permitted user engine 82 continuously, or at sampling intervals, updates the permitted user record PUDB for each analytical device P1A-P7A. In this way, each analytical device P1A - P7A comprises an accurate permitted user record PUDB(P1A) - PUDB(P7A), optionally stored in each analytical device P1A-P7A. This may reduce logon latency to the analytical devices P1A-P7A.

According to an embodiment, the permitted user record PUDB(P3A) for at least one analytical device P3A may be generated based partially on the time at which a user of the system was last seen at a location. According to embodiment, the permitted user record PUDB(P3A) for at least one analytical device P3A may be updated based on whether, or not, a shortest time duration of a path of a user from their last known location to the location of the at least one analytical device P3A has elapsed. Optionally, the shortest time duration of the path may be defined by the typical human ambulation speed between the last known location, and the location of the at least one analytical device P3A.

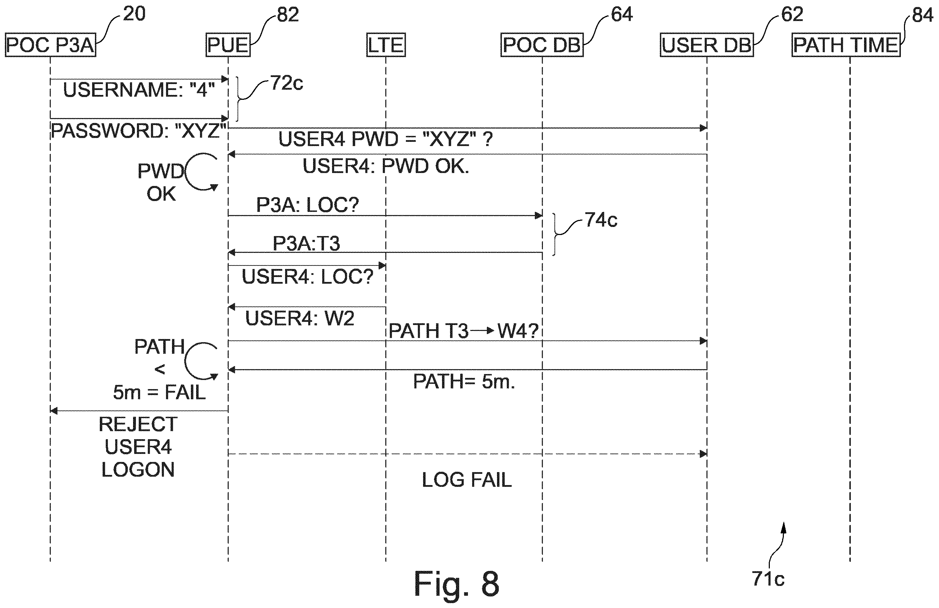

**FIG. 8** schematically illustrates a signalling diagram of location-based access control according to an embodiment. In particular, **FIG. 8** illustrates an unsuccessful logon to an analytical device based on a permitted user record PUDB owing to path time of a user between access control zones being below a permitted threshold.

In the example of **FIG. 8****,** the authentication and location lookup steps are as previously discussed. In this case, user "4" provides a correct password and is defined by the location tracking engine LTE in access control zone "W2". The analytical device P3A is defined in location "T3".

A variation of this example enables the permitted user engine 82 to interrogate the location time index 84. As noted above, the location time index 84 defines the approximate time of ambulation between a first location and the second location in the access-controlled facility 8. The location time index 84 reports to the permitted user engine 82 that the time to reach location "W4" from "T3" is in the range of five minutes. If a logon attempt to an analytical device P3A is made by a unauthorized user in the name of a user account "4", when the owner of the user account "4" has left location "W4", but not arrived at location "T3", then the permitted user engine 82 may infer that the correct user would not have time to arrive at the location of analytical device P3A from their previously known location.

The permitted user engine 82 therefore infers, based on information from the location time index 84, that user "4" cannot be present at the location of analytical device P3A, even though the logon credentials entered into analytical device P3A are those of user "4". In response, the permitted user engine 82 rejects the logon of user "4" to the analytical device P3A, and may optionally enter a log in the user database 62, or analytical device database 64, or may optionally alert a POC-DMS system as mentioned previously.

Usefully, this embodiment may make generation of the permitted user record PUDB more resilient when the network of the access control zones of the access controlled facility 8 is imperfect, incomplete, or has gaps that enable a user to be lost to the LTE for a period of time. A typical example would be a hospital spread out over a large, outdoor site where a user must walk between buildings in an area without access control.

According to an embodiment, the building information model database 66 may be used to automatically generate (parse) a connectivity model representing part of, or all of, the access-controlled facility 8. For example, the building information model database may contain floor plan, stairwell, lift, and other building access information stored in the IFC (Industry Foundation Classes) format as defined in ISO 16739 or "openBIM".

In an example, the building information model may be parsed into a connectivity graph model.

According to an embodiment, the method further comprises obtaining a connectivity graph model comprising one or more nodes and edges representing an access scheme of the access controlled facility, and mapping the location credential of the first user received from the location management system to the graph model.

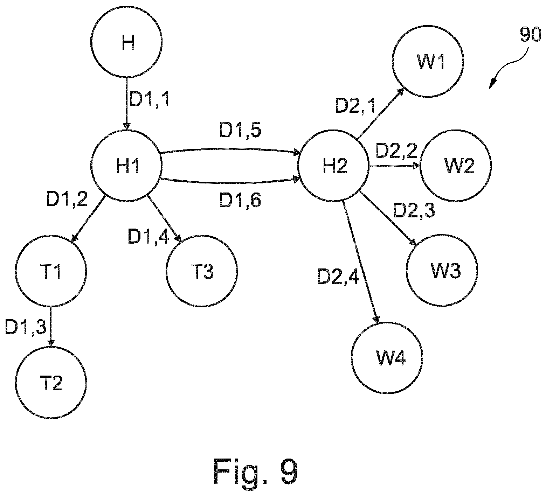

**FIG. 9** schematically illustrates a connectivity graph model of the access plan of the hospital illustrated in **FIG. 4****.**

The edges of the graph define an access portal between at least two access control zones. The vertices of the graph represent an access control zone, optionally containing a token representing the presence of an analytical device. The access portal labels "D x,y" of the edges, and the access control zone labels of the vertices map to the access plan of the hospital illustrated in **FIG. 4****.** The entry to the graph in this case is from the vertex H, although, of course, many different entrances and exits to the graph could be modelled.

It is not necessary that the connectivity graph perfectly defines an access control facility 8. However, to the extent that the generated connectivity graph varies from the real-life access-controlled facility 8, minor inaccuracies may be introduced into the logon control scheme discussed in this specification. However, a hospital administration may take the view that even if the connectivity graph contains minor inaccuracies compared to the genuine floor plan of the access controlled facility 8, the techniques discussed in this specification can still significantly improve the probability (accuracy) of analytical device access control, compared to the absence of such a system.

Optionally, the data processing agent 70 may enforce different access policies on different users.

A first user may be provided with access to different access control zones compared to a second user. Accordingly, the building information model may comprise a plurality of connectivity graphs, one for each security policy in existence. Optionally, the permitted user engine 82 is configured to update the permitted user record PUDB(P3A) of at least one analytical device P3A based, additionally, upon the connectivity graph matching the security policy of a first user.

Usefully, applying a graph as a model of the access control zones of the hospital enables a path between two vertices to be efficiently and quickly generated. Furthermore, time weightings can be applied to the edges to enable average ambulation times to be calculated. Additionally, a graph is visually intuitive and may be displayed on the graphical user interface of a POC-DMS control system controlling a large variety of analytical devices P1A-P7A.

In addition, a graph data structure may be manually edited, by clicking and dragging icons on the screen, to enable different floor plans or floor plan changes to be easily adopted or modelled. As will be shown, once a graph representation of a floor plan of an access control facility 8 has been derived, it may be flexibly used to determine analytical device access control policies.

According to an embodiment, the method further comprises labelling only the present node in the connectivity graph model as representing the current location of the first user; and updating the permitted user record PUDB to remove the first user from the permitted user record PUDB so that an analytical device at a location of the access controlled facility represented by an unlabelled node of the connectivity graph model cannot be accessed by a second user using the same user logon credentials as the first user.

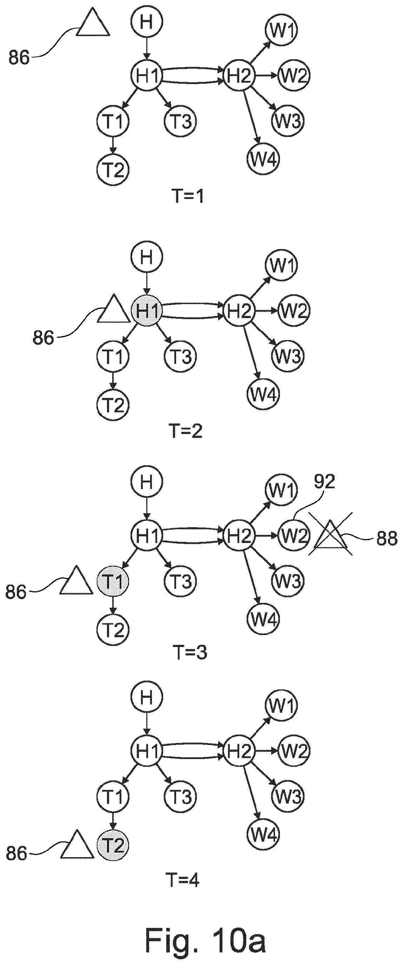

**FIG. 10a** schematically illustrates an example of a user represented in the connectivity graph model at different times. A legitimate user 86 enters the hospital via the hallway H, progressing through the lower hallway HI to the testing facility T2, via the testing facility T1. (The edge labels are not shown for reasons of clarity on the graph representations of **FIG.10a****,** but they are the same as those in the enlarged graph representation of **FIG.9**).

In a first option, one node (vertex) of the graph may be labelled to denote the current location of the legitimate user 86.

When a unauthorized user 88 (such as an untrained user, or a user who has misappropriated the password of legitimate user 86) attempts to use analytical device P7A in ward W2, a permitted user record PUDB(7) associated with the analytical device P7A does not comprise a record of a legitimate user 86, because legitimate user 86 has not been identified as being present in the access control zone of ward W2 (the node of the graph has not been labelled to denote the current location of the legitimate use 86). Therefore, the unauthorized user 88 is not able to logon to the analytical device P7A.

According to an embodiment, the method further comprises labelling one or more nodes in the connectivity graph model subsequent to a node of the connectivity graph model representing the current location of the first user, and updating the permitted user record PUDB to remove the first user from the permitted user record PUDB so that an analytical device at a location of the access controlled facility represented by an unlabelled node of the connectivity graph model cannot be accessed by a second user using the same user logon credentials as the first user.

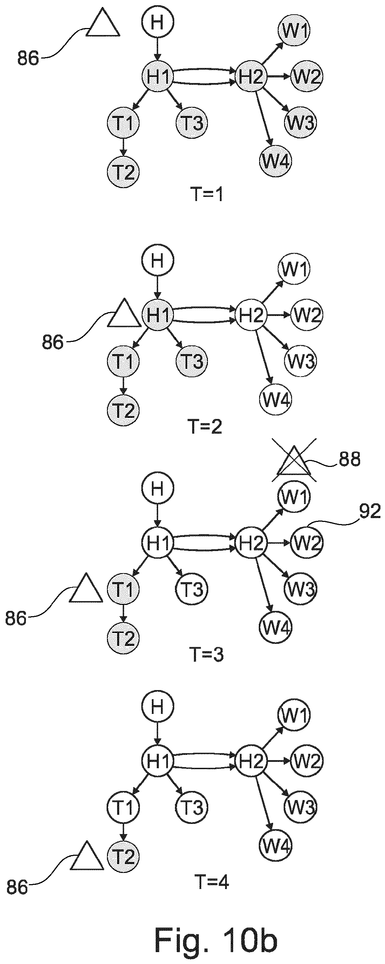

**FIG. 10b** schematically illustrates another example of a user represented in the connectivity graph model at different times.

In the second option, all nodes (vertices) of the connectivity graph may be labelled. In this representation, a labelled node of the connectivity graph downstream from the current position of the legitimate user 86 represents an access control zone of the access controlled facility 8 that it is possible for the legitimate user 86 to eventually reach, starting from their current position. As the legitimate user 86 advances through the access control facility 8, nodes of the connectivity graph are unlabelled to represent locations that it is not possible for the legitimate user 86 to access based on their current location.

At T = 1 it is possible for the legitimate user 86 to be in all access-controlled areas. Accordingly, the permitted user database PUDB of every analytical device in the hospital P1A-P7A contains an entry of the legitimate user 86.

For example, between T = 2 and T = 3, the legitimate user moves from hallway HI into testing room T1. This removes the possibility to use the stairwell D 1,5 or lift D 1,6 to access the upper floor of the hospital, and hence the upper floor of the hospital is unlabelled or pruned from the graph. In practical terms, as a node of the connectivity graph is unlabelled, an entry is removed from the permitted user database PUDB of an analytical device located in the access control zone corresponding to the location of the unlabelled node of the connectivity graph.

Therefore, when an unauthorized user 88 attempts to use the analytical device P4A in W1, using the misappropriated logon details of legitimate user 86, the permitted user database PUDB(4A) of the analytical device P4A does not contain an entry in respect of legitimate user 86, and it is not possible for the unauthorized user 88 to logon to the analytical device P4A.

In the foregoing embodiment, the successive pruning of the connectivity graph represents a reductive approach that may be more suitable to situations in which the connectivity graph is not a fully accurate representation of the hospital floor plan.

According to an embodiment, the location credential defines the presence of the user in either (i) a first, insecure, location that does not contain the analytical device, or (ii) in a second, secure, location that does contain the analytical device.

As system complexity increases, and there are more rooms, users, and analytical devices to manage the data pressing agent 70, the sampling interval for updating the permitted user records may lead to an increase in communication overhead. An acceptable sampling interval for each permitted user record PUDB update may, for example, be related to the speed of human ambulation through a building, the present location of the user in a PUDB, and the present location of respective PUDB, as will be discussed subsequently.

According to an embodiment, the location management system obtains location credentials from a swipe or RFID card access system, an iris scanning system, a facial recognition system, a QR or barcode based access system, a Wiegand access system, a PIN access system, a photo-ID system, an elevator control system, and/or a wireless networking tracking system.

Other access control techniques that monitor access, or control access between first and second access control zones of a building, or outdoor area, may be used with the location management system 68 without departure from the teaching of this specification. Furthermore, any combination of signals or information from the previously listed location management (access control) approaches may be combined to provide a more accurate estimate of the location of a user in an access-controlled facility 8.

In addition, the functionality of the location management system 68 could be provided by a location server monitoring the location of a user using GPS, preferably via the mobile phone of the user. For example, the location management system 68 could interface to a location tracking server of a 3G, 4G, or 5G mobile telephone network that monitors user location in combination with a global positioning system (GPS) function of user handsets. The resolution of GPS is enough to track the location of a user across a hospital campus. Therefore, the access control zones could be defined partly by whether, or not, the user has entered or exited a building using GPS monitoring.

According to an embodiment, the computer-implemented method 71a-d further comprises:
Obtaining a certification credential 60b of the first user of the analytical device from a user certification database 60;
obtaining certification requirement data of the analytical device from an analytical device certification requirement database 64; and
permitting the first user to logon to the analytical device if the certification credential of the first user accords with the certification requirement data, or denying the first user the ability to logon to the analytical device P1A-P7A if the certification credential of the first user does not accord with the certification requirement data.

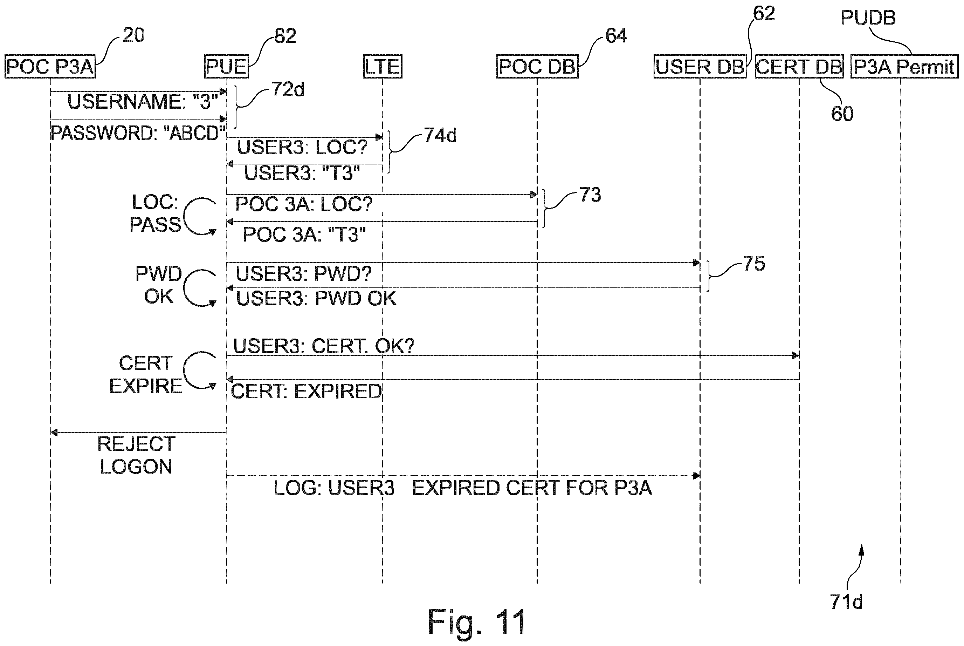

**FIG. 11** schematically illustrates a signalling diagram of location-based access control according to an embodiment. In particular, **FIG. 11** illustrates an unsuccessful logon to an analytical device based on a permitted user record owing to an unsatisfactory certification condition.

In particular, receiving 72 a user logon credential, receiving 74 location credential data of the user, receiving 73 location data of the analytical device P3A from the analytical device database 64, and verifying the user password 75 in the user database 62 are as described in relation to at least **FIG. 6** above will not be repeated for the purpose of brevity.

According to this example, the permitted user engine 82 interrogates, using, for example, the username "3", the certification database 62. In this example, the certification database 62 returns to the permitted user engine 82 the information that a certificate of user "3" for use on analytical device P3A (in other words, the same analytical device that user "3" is attempting to logon to) has expired. For this reason, the permitted user engine 82 rejects the logon attempt to analytical device P3A. Optionally, the permitted user engine 82 transmits a log entry to, for example, the certification database 60, the user database 62, or the analytical device database 64.

According to an embodiment, the computer-implemented method further comprises:
detecting, via the user certification database, that the certification status of the first user has been changed, such that the first user is no longer certified to logon, or remain logged on, to the analytical device; and
removing the first user from the permitted user record PUDB of the analytical device.

Accordingly, the permitted user engine 82 may dynamically update the permitted user record PUDB based on the certification state of the user as defined in the certification database 60. For example, if the permitted user engine 82 becomes aware that a given certificate of a given user has expired, and if that certificate is required for operating analytical device P3A, the user may be removed from the permitted user record PUDB of analytical device P3A.

According to an embodiment, the analytical device P IA-P7A is configured to analyse biological samples to identify a biomarker of a medical condition.

For example, the analytical device P1A-P7A may be configured to perform one or more of the tests on a biological sample obtained from a patient.

A further specific example will now be discussed. This example does not map directly to the illustration of **FIG 4****.**

Consider a hospital with two floors, and two wings (West Wing and East Wing) on each floor. A first user has access rights to all access control zones of the hospital. The hospital has a first analytical device A on the first floor of the West Wing, and a second analytical device B on the second floor in the East Wing. The hospital houses a point-of-care IT data management system (POC-DMS) executing a data processing agent 70 as discussed herein. The POC-DMS 40A controls access to the first and second analytical devices users training records and their job description in hospital. The hospital also comprises a door access control system that controls the opening of doors between every access control zone of the hospital.

At least the following example scenarios may be handled by the computer implemented method according to the first aspect or its embodiments:
A) "User has a valid certification and enters the area of the analytical device A":
   When a user enters the access control zone containing device A, using an access badge, a message is sent to the POC-DMS. The POC-DMS combines that information with the fact that the user is certified to use analytical device A. A message is sent from the POC-DMS to analytical device A so that the user can log in to analytical device A.
B) "User has a valid certification and leaves the area of the analytical device A"
   When a user leaves the access control zone containing analytical device A, and a message is sent from a gate management system to the POC-DMS confirming this, the POC-DMS combines the information from the gate management system with the fact that the user is certified to use analytical device A. A message is sent to analytical device A to remove the user from the list of permitted users of the analytical device A.
C) "User does not have a valid certification for analytical device B and enters the area of analytical device B"
   When the user enters the access control zone of the analytical device B using their badge, a message is sent from the access control system to the POC-DMS. The POC-DMS combines that information with the fact that the user is not certified to use analytical device B. A message is not sent to analytical device B updating its permitted user list, and the user cannot logon to analytical device B.
D) "User is not trained to use analytical device B and leaves the area of analytical device B"
   When the user leaves the access control zone of analytical device B, a message is sent from the access control system to the POC-DMS. The POC-DMS combines the information with the fact that the user is not certified to use analytical device B. No information is sent to analytical device B.

According to a second aspect, there is provided an apparatus 40 configured to control user access to an analytical device P1A-P7A based on a location of a user relative to the analytical device. The apparatus comprises:
- a communications interface 54; and
- a processor 47 operably coupled to the communications interface 54.

The communications interface 54 is configured to receive a first location credential LTE of a first user of an analytical device from a location management system 68 of an access controlled facility 8, wherein the first location credential LTE at least partially defines a current location of the first user of the analytical device P1A-P7A.

The processor 47 is configured to update a permitted user record PUDB(i) associated with the analytical device P1A-P7A based on the first location credential LTE of the first user.

The communications interface 54 is configured to receive a user logon credential entered into the analytical device P1A-P7A as part of a logon process of the analytical device.

The processor 47 is configured to permit a logon to the analytical device if the user logon credential entered into the analytical device accords with the permitted user record PUDB.

According to a third aspect, there is provided a system 10A for controlling user access to an analytical device P1A-P7A based on a location of a user relative to the analytical device for analytical device management 40A. The system 10A comprises:
- one or more analytical devices P1A-P7A, optionally configured to analyse patient samples;
- a location management system 68 configured to detect when a user leaves or enters the vicinity of the one or more analytical devices P1A-P7A;
- an apparatus 40 according to the second aspect which, in operation, performs the method of the first aspect, or its embodiments; and
- a communication network 10A, 10B configured to communicatively connect the one or more analytical devices P1A-P7A, the location management system 68, and the apparatus 40 according to the second aspect.

According to the fourth aspect, there is provided a computer program element comprising computer-readable instructions for controlling an apparatus according to the second aspect which, when being executed by a processing unit of the apparatus, is configured to perform the method steps of the first aspect, or its embodiments.

According to the fifth aspect, there is provided a computer readable medium or signal having stored, or encoded thereon, the computer program element according to the fourth aspect.

A skilled person will appreciate that the embodiments of the apparatus according to the second aspect may be provided by configuring the processor of the apparatus to perform processing operations according to the embodiments of the computer implemented method according to the first aspect, and that this specification is a disclosure of such apparatus embodiments of the second aspect.

Having described the present disclosure in detail and by reference to specific embodiments thereof, it will be apparent that modifications and variations are possible without departing from the scope of the disclosure defined in the appended claims. More specifically, although some aspects of the present disclosure are identified herein as preferred or particularly advantageous, it is contemplated that the present disclosure is not necessarily limited to these preferred aspects of the disclosure.

## Claims

1. A computer implemented method (71a-d) for controlling user access to an analytical device (P1A-P7A) based on a location of a user (86) relative to the analytical device (P1A-P7A), the method comprising:
receiving (72) a user logon credential of a first user entered into an analytical device as part of a logon process of the analytical device;
receiving (74) a first location credential of a first user of the analytical device (P1A-P7A) from a location management system (68) of an access controlled facility (8), wherein the first location credential at least partially defines a current location of the first user of the analytical device;
updating (76) a permitted user record (PUDB) associated with the analytical device based on the first location credential of the first user; and
permitting (78) a logon to the analytical device if the received user logon credential entered into the analytical device accords with the permitted user record (PUDB), as updated based on the first location credential.

2. The computer implemented method (71a-d) according to claim 1, further comprising:
obtaining a certification credential of the first user of the analytical device (P1A-P7A) from a user certification database (60);
obtaining certification requirement data of the analytical device from an analytical device certification requirement database (64); and
permitting the first user to logon to the analytical device if the certification credential of the first user accords with the certification requirement data, or denying the first user the ability to logon to the analytical device if the certification credential of the first user does not accord with the certification requirement data.

3. The computer implemented method (71a-d) according to one of the preceding claims, further comprising:
detecting, via the location management system (68), that the first user has left an access controlled area containing the analytical device based on a second received location credential; and
removing the first user from the permitted user record (PUDB).

4. The computer implemented method (71a-d) according to one of the preceding claims, further comprising:
detecting, via the user certification database (60), that the certification status of the first user has been changed, such that the first user is no longer certified to logon, or remain logged on, to the analytical device (P1A-P7A); and
removing the first user from the permitted user record (PUDB).

5. The computer implemented method (71a-d) according to one of the preceding claims,
wherein the permitted user record (PUDB) is hosted by the analytical device, and
updating the permitted user record to define that the first user is permitted, or not permitted, to logon to the analytical device based on the location credential of the first user.

6. The computer implemented method (60) according to one of the preceding claims,
wherein the analytical device is configured to analyse biological samples to identify a biomarker of a medical condition.

7. The computer implemented method (71a-d) according to one of the preceding claims, further comprising:
obtaining a connectivity graph model (90) comprising one or more nodes and edges representing an access scheme of the access-controlled facility (8);
mapping the location credential of the first user received from the location management system (68) to the connectivity graph model (90).

8. The computer implemented method (71a-d) according to claim 7, further comprising:
labelling one or more nodes in the connectivity graph model (90) subsequent to a node of the connectivity graph model representing the current location of the first user; and
updating the permitted user record (PUDB) to remove the first user (86) from the permitted user record so that an analytical device at a location of the access controlled facility represented by an unlabelled node of the connectivity graph model cannot be accessed by a second user using the same user logon credentials as the first user.

9. The computer implemented method (71a-d) according to claim 8, further comprising:
labelling only a present node in the connectivity graph model (90) as representing the current location of the first user; and
updating the permitted user record (PUDB) to remove the first user from the permitted user record so that an analytical device at a location of the access controlled facility represented by an unlabelled node (92) of the connectivity graph model cannot be accessed by a second user (88) using the same user logon credentials as the first user (86).

10. The computer implemented method (71a-d) according to one of the preceding claims,
wherein the location credential defines the presence of the user in either (i) a first, insecure, location that does not contain the analytical device, or (ii) in a second, secure, location that does contain the analytical device.

11. The computer implemented method (71a-d) according to one of the preceding claims,
wherein the location management system obtains location credentials from a swipe or RFID card access system, an iris scanning system, a QR or barcode based access system, a Wiegand access system, a PIN access system, a photo-ID system, an elevator control system, and/or a wireless network tracking system.

12. An apparatus (40) configured to control user access to an analytical device based on a location of a user relative to the analytical device, wherein the apparatus comprises:
- a communications interface (54); and
- a processor (47) coupled to the communications interface;
wherein the communications interface is configured to receive a user logon credential of a first user entered into the analytical device as part of a logon process of the analytical device; and
wherein the communications interface is configured to receive a first location credential LTE of a first user (86) of an analytical device (P1A-P7A) from a location management system (68) of an access controlled facility, wherein the first location credential at least partially defines a current location of the first user of the analytical device;
wherein the processor is configured to update a permitted user record (PUDB) associated with the analytical device based on the first location credential of the first user;
wherein the processor is configured to permit a logon to the analytical device if the user logon credential entered into the analytical device accords with the permitted user record (PUDB), as updated based on the first location credential.

13. A system (10A, 10B) for controlling user access to an analytical device based on a location of a user relative to the analytical device for analytical device management comprising:
- one or more analytical devices (P1A-P7A);
- a location management system (68) configured to detect when a user leaves or enters the vicinity of the one or more analytical devices;
- an apparatus (40) as defined in claim 12 which, in operation, performs the method of claims 1 to 11; and
- a communication network (16A) configured to communicatively connect the one or more analytical device, the location management system, and the apparatus.

14. A computer program element comprising computer-readable instructions for controlling an apparatus according to claim 13 which, when being executed by a processing unit of the apparatus, is configured to perform the method steps of one of claims 1 to 12.

15. A computer readable medium or signal having stored, or encoded thereon, the computer program element of claim 14.
